Europäisches Patentamt
European Patent Office
Office européen des brevets

(11) Publication number: 0 346 068 A2

# EUROPEAN PATENT APPLICATION

(21) Application number: 89305690.3

(22) Date of filing: 06.06.89

(51) Int. Cl.4: C07K 7/06, C07K 7/16, A61K 37/64

Claims for the following Contracting States: ES + GR

(30) Priority: 07.06.88 US 203389

(43) Date of publication of application: 13.12.89 Bulletin 89/50

(84) Designated Contracting States: AT BE CH DE ES FR GB GR IT LI LU NL SE

(71) Applicant: SMITHKLINE BECKMAN CORPORATION
One Franklin Plaza P O Box 7929
Philadelphia Pennsylvania 19103(US)

(72) Inventor: Huffman, William Francis
40 Crest Avenue
Malvern Pennsylvaia 19355(US)
Inventor: Kinter, Lewis Boardman
200 East Eagle Road
Havertown Pennsylvania 19083(US)
Inventor: Moore, Michael Lee
417 South Jackson Street
Media Pennsylvania 19063(US)
Inventor: Winslow, Christine Ruth
325 Headhouse Court
Wayne Pennsylvania 19087(US)

(74) Representative: Waters, David Martin, Dr. et al
Smith Kline & French Laboratories Ltd.
Patent Department Mundells
Welwyn Garden City Hertfordshire AL7 1EY(GB)

(54) D-Cys6 vasopressin antagonists.

(57) Compounds of the Formula I:

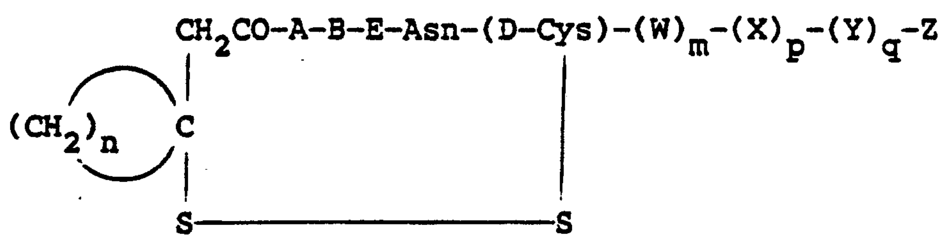

Formula I

are described, in which:
A is D or L isomer of Phe, Phe(4′-Alk), Ile, Cha, Tyr, or Tyr(O-Alk);
B is Phe, Phe(4′-Alk), Tyr(O-Alk), Ile or Tyr;
E is Val, Ile, Abu, Chg, Gln, Lys, Cha, Nle, Leu, Ala or Gly;
n is 4 or 5;
m, p and q are each 0 or 1;
W is D or L isomer of Pro, Arg, HArg, N-MeArg, Lys or Orn;
X is D or L isomer of Arg, Lys, Orn or Gln or when m and q are 1, X may be Gly;
Y is D or L isomer of Arg, Lys, Orn, Ser, Gln, Tyr or Ala;

EP 0 346 068 A2

Xerox Copy Centre

Z is NH-$(CH_2)_t$-NHR, or a D or L isomer of Arg-$NH_2$, Lys-$NH_2$ or Orn-$NH_2$ or when at least one of m, p and q is 1, Z may be NHR′ or OH;
R is H or C(=NH) $NH_2$;
R′ is H or Alk;
t is 2-6; and
Alk is alkyl having 1-4 carbon atoms; or a pharmaceutically acceptable acid addition salt, or lower alkyl or benzyl ester thereof. Processes for their production, pharmaceutical compositions comprising them and their use as medicaments are described.

# D-CYS[6] VASOPRESSIN ANTAGONISTS

## Field of the Invention

This invention relates to cyclic peptide compounds, characterized by D-Cys unit in the 6-position, which exhibit vasopressin antagonist activity and are substantially devoid of vasopressin agonist activity. This invention further relates to pharmaceutical compositions and methods for producing vasopressin antagonist activity without substantial agonist activity in patients in need thereof.

## Background of the Invention

The compounds of this invention exhibit $V_1$ and/or $V_2$ vasopressin antagonist activity without substantial agonist activity. Vasopressin is known to contribute to the antidiuretic mechanism of actions within the kidney. The action of these compounds antagonizes that of the natural anti-diuretic hormone (ADH) so as to cause the body to excrete water.

Manning et al., Nature 308:652 (1984) have described the following compound

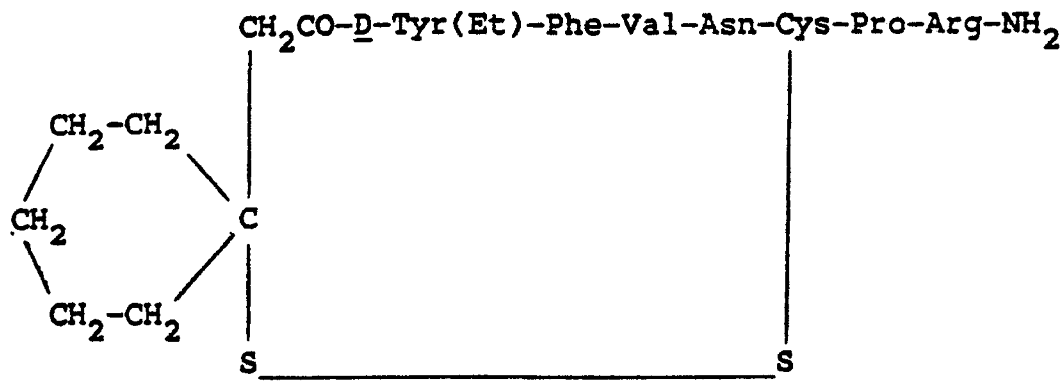

**FORMULA A**

as having potent vasopressin antagonist activity. Also, this compound is reported as being devoid of detectable vasopressin agonist activity in vivo in rats. In man, however, this compound demonstrated only agonist activity, Dubb et al., Kidney Int. 31:267 (January 1987). A new test in dogs now shows that this compound does produce agonist activity.

The compounds of the present invention are distinguished over the above Manning et al. compound (Formula A) in that the cysteine unit in the 6-position is the D-isomer and these compounds, while having vasopressin antagonist activity, are substantially devoid of agonist activity as shown by the test in dogs.

Certain 6-(D-Cys) compounds having vasopressin antagonist activity are described by Manning et al. in U.S. Patent No. 4,491,577. All of those compounds have a Pro-Z-Gly-$NH_2$ tail. The compounds in the present invention do not have a terminal glycine unit in a tripeptide tail.

The effect of removal or replacement of amino acid groups in the tail of the peptide has been reported for compounds having the L-isomer of cysteine in the 6-position. Manning et al., Nature 308:652 (1984) and U.S. Patent No. 4,469,679 have disclosed that the terminal glycine unit at the 9-position of certain 1-($\beta$-mercapto-$\beta$,$\beta$-cyclopentamethylene propionic acid) vasopressin compounds can be removed or replaced by L- or D-Ala, Ser or Arg without necessarily affecting the activity as vasopressin antagonists. U.S. Patent Nos. 4,481,194 and 4,481,193 have disclosed that removing proline at position 7 or both proline and glycine at positions 7 and 9 from the structures of vasopressin antagonist compounds will produce compounds which retain substantial, but somewhat reduced, antagonist activity.

The interesting activity of the 6-(D-Cys) vasopressin antagonists of this invention was only discovered

after the dog test became available to demonstrate that these compounds do not produce substantial agonist activity. The rat test which was used to test absence of vasopressin agonist activity.

## Description of the Invention

The compounds of this invention are illustrated by the following structural formula:

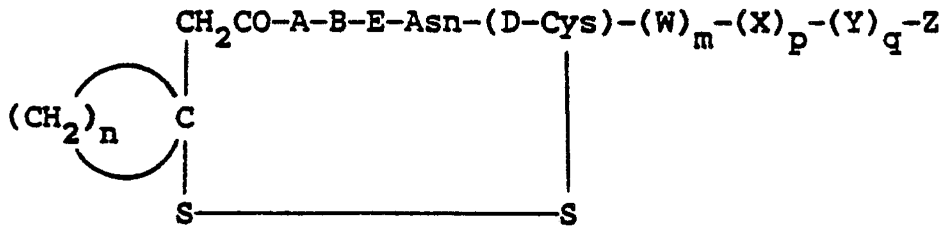

**Formula I**

in which:
A is D or L isomer of Phe, Phe(4′-Alk), Ile, Cha, Tyr or Tyr(O-Alk);
B is Phe, Phe(4′-Alk), Tyr(O-Alk), Ile or Tyr;
E is Val, Ile, Abu, Chg, Gln, Lys, Cha, Nle, Leu, Ala or Gly;
n is 4 or 5;
m, p and q are each 0 or 1;
W is D or L isomer of Pro, Arg, HArg, N-MeArg, Lys or Orn;

X is D or L isomer of Arg, Lys, Orn or Gln, or when m and q are 1, X may be Gly;
Y is D or L isomer of Arg, Lys, Orn, Ser, Gln, Tyr or Ala;
Z is NH-$(CH_2)_t$-NHR, or a D or L isomer of Arg-$NH_2$, Lys-$NH_2$ or Orn-$NH_2$ or when at least one of m, p and q is 1, Z may be NHR′ or OH;
R is H or C(=NH)-$NH_2$;
R′ is H or Alk;
t is 2-6; and
Alk is alkyl having 1-4 carbon atoms; or a pharmaceutically acceptable acid addition salt, or lower alkyl or benzyl ester thereof.

A subgeneric group of compounds of this invention are compounds of Formula I in which:
A is D-Tyr or D-Tyr(Et);
B is Phe;
E is Val;
m and p are 1 and q is 0;
W is a D or L isomer of Pro, Arg or N-MeArg;
X is a D or L isomer of Arg or Gly; and
Z is NHR or a D or L isomer of Arg-$NH_2$.

Particular $(W)_m$-(X)-$_p$-$(Y)_q$-Z tails are Pro-Arg-$NH_2$, D-Arg-Arg-NH2, Arg-Arg-$NH_2$, Arg-D-Arg-$NH_2$, D-Arg-D-Arg-$NH_2$, and N-MeArg-Arg-$NH_2$. Other tails are Pro-Arg-NH$(CH_2)_2NH_2$, Pro-NH$(CH_2)_4$ NHC(=NH)-$NH_2$, Arg-$NH_2$, and Arg-Gly-Arg-$NH_2$.

Particular compounds of this invention are [1-(β-mercapto-β,β-cyclopentamethylene-propionic acid)-2-(O-ethyl)-D-tyrosine-4-valine-6-D-cysteine-9 desglycine]-vasopressin,
[1-(β-mercapto-β,β-cyclopentamethylenepropionic acid)-2-(O-ethyl)-D-tyrosine-4-valine-6-D-cysteine-7-D-arginine-9-desglycine]-vasopressin,
[1-(β-mercapto-β,β-cyclopentamethylenepropionic acid)-2-(O-ethyl)-D-tyrosine-4-valine-6-D-cysteine-7-N-methylarginine-9-desglycine]-vasopressin,
[1-(β-mercapto-β,β-cyclopentamethylenepropionic acid)-2-(O-ethyl)-D-tyrosine-4-valine-6-D-cysteine-7-arginine-9-desglycine]-vasopressin,
[1-(β-mercapto-β,β-cyclopentamethylenepropionic acid)-2-(O-ethyl)-D-tyrosine-4-valine-6-cysteine-7-arginine-8-D-arginine-9-desglycine]-vasopressin,
[1-(β-mercapto-β,β-cyclopentamethylenepropionic acid)-2-(O-ethyl) D-tyrosine-4-valine-6-D-cysteine-7-desproline-9-desglycine]-vasopressin.

Also included in this invention are addition salts, complexes or prodrugs, such as esters of the compounds of this invention when Z is OH, and especially the nontoxic, pharmaceutically acceptable acid addition salts. The acid addition salts are prepared in standard manner in a suitable solvent from the parent compound and an excess of an acid, such as hydrochloric, hydrobromic, sulfuric, phosphoric, acetic, maleic, succinic, ethane-disulfonic or methanesulfonic acid. The ester derivatives of the acid form of the end products, such as the methyl, ethyl or benzyl esters, are prepared as known in the art.

In the description herein and in the claims, the nomenclature common in the art of peptide and vasopressin chemistry is used. When no configuration is noted, the amino acid unit is in the L, or naturally occurring, form. In certain structural formulas, the thio members, for example in Cys, are added for clarity.

The peptide art designations contained herein include the following: Pmp, β-mercapto-β,β-cyclopentamethylenepropionic acid; Abu, α-amino-n-butyric acid; Chg, cyclohexylglycine; Cha, cyclohexylalanine; Cys, cysteine; Gln, glutamic acid amide or glutamine; Gly, glycine; Tyr, tyrosine; Phe, phenylalanine; Val, valine; Ile, isoleucine; Nle, norleucine; Leu, leucine; Ala, alanine; Lys, lysine; Arg, arginine; HArg, homoarginine; Orn, ornithine; Ser, serine; Pro, proline; Tos, tosylate; Bzl, benzyl; MBzl, p-methoxybenzyl; Boc, t-butoxycarbonyl; ClZ, 2-chlorobenzyloxycarbonyl; DMAP, dimethylaminopyridine; DCC dicyclohexylcarbodiimide; HBT, hydroxybenzotriazole; HF, hydrogen fluoride; TFA, trifluoroacetic acid; DMF, dimethylformamide.

"Alk" represents a lower alkyl of 1 to 4 carbons. Such alkyl substituents include methyl, ethyl, n-propyl, isopropyl or butyl. The preferred alkyl substituent is ethyl.

When the term "vasopressin" is used, it means L-arginine vasopressin unless otherwise modified to indicate a D-arginine, leucine, etc. containing vasopressin.

The compounds of Formula I are prepared by cyclizing a linear peptide by means of the two mercapto groups at the cysteine unit at position 6 and the β-mercapto-β,β-cyclopenta(or cyclotetra)-methylenepropionic acid at position 1. The cyclization reaction occurs in the presence of a mild oxidizing agent capable of oxidizing a mercaptan to a disulfide. Thus, linear peptides of the following formula are oxidized:

$R^a$-A-B-E-Asn-(D-Cys)-$(W)_m$-$(X)_p$-$(Y)_q$-Z
Formula II

in which $R^a$ is β-mercapto-β,β-cyclopenta(or cyclotetra)methylenepropionic acid and A,B,E,W,X,Y,Z,m,p and q are as defined in Formula I, with the mercapto groups being members of the $R^a$ and D-Cys units.

The oxidation is carried out using, for example, an excess of an alkali metal ferricyanide, such as potassium or sodium ferricyanide. A suitable unreactive solvent, preferably an aqueous solvent at neutral pH, about 7-7.5, is used. The reaction is carried out at ambient temperature or lower until the reaction is substantially complete. Preferably, low concentrations of the linear peptide dimercaptan and the oxidizing agent are used, such as about 0.01 to 0.1 molar concentration of oxidizing agent in several liters of aqueous solution to cyclize about 1-5 grams of dimercaptan.

Other mild oxidation agents having an oxidation potential roughly equivalent to ferricyanide may also be used for the ring closure reaction such as passing oxygen through the reaction solution for several days. In addition, iodine in methanol can be used on the unprotected peptide or the acetamidomethyl sulfur-protected derivatives. Cyclization also occurs when a displaceable, thiol-protective group such as that at the mercaptan group of the $R^a$ unit is displaced intramolecularly.

Of course, one skilled in the art would recognize that when an interfering reaction site is present in the structure of the starting material of Formula II, the linear mercaptan starting material may have common protective groups temporarily present at the various amino acid units.

The compounds of Formula I in which Z is $NH(CH_2)_tNHR$ are conveniently prepared by the following procedure:

$R^a$-A-B-E-Asn-(D-Cys)-$(W)_m$-$(X)_p$-$(Y)_q$(OH) + $NH_2(CH_2)_tNHR$
|                              |
S——————————————S

as described in examples herebelow. The procedure is described by Callahan et al., U.S. Patent No.

4,543,349.

The peptides of Formula I are conveniently isolated by acidifying the aqueous oxidation mixture, such as with glacial acetic acid, and passing the reaction mixture over an ion-exchange chromatographic column, for example over a weakly acid, acrylic resin column with elution using buffered base, or by gel filtration over a bead-formed gel prepared by cross-linking dextran with epichlorohydrin.

The intermediates of Formula II, in free or protected form, are conveniently prepared using solid-phase methods of peptide synthesis as discussed in Manning et al., J. Med. Chem. 25:46 (1982). A commercial benzhydrylamine support resin (BHA) is used to prepare the amide end products of Formula I, and a chloromethyl support resin (CMR) is used to prepare the acid compounds of Formula I, i.e. in which Z is OH. Solution or enzymatic synthetic methods can also be used.

The peptide chain of the linear peptides is usually built up, stepwise, proceeding from the last unit (in the 7,8,9 or 10 positions) and working toward the R unit. Each unit is properly protected as known in the peptide art and as described below. The sequence of step-reactions is conveniently carried out in a Beckman 990B peptide synthesizer or its equivalent without isolation of each intermediate peptide. The details of the procedure are in the examples presented hereinafter.

The various amino acids, which are consecutively added to the resin-supported chain, are protected as known in the art. For example, the t-butoxycarbonyl protecting group may be used for an amino group, especially at the α-position; an optionally substituted benzyl or acetamidomethyl for the mercapto groups at the $R^a$- or D-Cys units; tosyl for the Arg, HArg or N-MeArg units; and an optionally substituted benzyloxycarbonyl for the Tyr or Lys units. The protective groups are, most conveniently, those which are not easily removed by using mild acid treatment. It is preferable to use protective groups that are removed using sodium-liquid ammonia. Other protective groups are known to the art, such as those noted in "Protective Groups in Organic Chemistry" by J.F.W. Mcomie (Plenum 1973).

The protected linear peptide intermediate is split from the carrying resin matrix, for example, by using ammonia in an aqueous miscible solvent, and then is treated to remove the protective groups, such as with sodium-liquid ammonia. This procedure gives the amide derivative of the linear peptide intermediate.

More conveniently, the two steps are combined by treating the resin supported peptide with anyhdrous hydrogen fluoride using a suitable carbonium ion scavenger, such as anisole, to give the linear peptide intermediate of Formula II.

The $R^a$ intermediate, protected with benzyl on the mercapto group, is prepared by reacting ethyl cyclohexylidene (or cyclopentylidene) acetate with benzylmercaptan by the procedure described by Yim and Huffman, Int. J. Peptide Protein Res. 21:568 (1983) for the preparation of β-(S-benzylmercapto)-β,β-cyclopentamethylenepropionic acid. The product obtained in this preparation is purified by recrystallization from a suitable solvent such as hexane.

The compounds of Formula I have $V_1$ and/or $V_2$ vasopressin antagonist activity. Vasopressin is known to contribute to the anti-diuretic mechanism of action within the kidney. When the action of these compounds antagonizes that of the natural anti-diuretic hormone (ADH), the body excretes water due to an increased permeability of the terminal portions of the renal tubule. The mechanism of action is at the vasopressin receptors ($V_2$-receptors) located on the plasma membrane of certain renal epithelial cells. The most notable pharmacodynamic effect of the ADH antagonists of this invention is that of a water diuretic, or aquaretic, rather than of a natriuretic such as hydrochlorothiazide.

$V_2$-antagonistic activity toward the natural anti-diuretic hormone (anti-ADH activity) is determined in vitro, in the medullary tissue of hog or human kidneys and, in vivo, in the hydropenic rat. The in vitro assay procedures for vasopressin stimulated adenylate cyclase activation or vasopressin binding activity are described by F. Stassen et al., J. Pharmacology and Experimental Therapeutics 223:50-54 (1982). $V_1$-antagonistic activity is determined by procedures using the rat thoracic aorta issue and plasma membranes of rat liver. These procedures are described in the noted Stassen publication and in a publication at the 1st International Conference on Diuretics, Miami, Florida, March (1984). Oxytocin antagonism is determined as described by W. Sawyer et al., Endocrinology 106:81 (1979).

A patient suffering, from the syndrome of inappropriate antidiuretic hormone condition or from an undesirable edematous condition is a target for the compounds of this invention. Examples of clinical conditions for which the compounds of this invention may be used include hypertension, hepatic cirrhosis, hyponatremia, congestive heart failure or a component, such as edema, or any traumatic condition resulting from serious injury or disease.

The second group of vasopressin receptor sites are the vascular pressor sites ($V_1$-receptors) within the cardiovascular system itself. These may also be antagonized by the compounds of this invention.

The compounds of this invention are also oxytocin antagonists and as such are useful to prevent premature labor and in the treatment of primary dysmenorrhea.

The compounds of this invention, therefore, are used especially to induce anti-hypertensive, anti-oxytocic or diuretic activity in patients in need of such treatment. The compounds are administered internally, parenterally, buccally or by insufflation, in a nontoxic but effective quantity, preferably dispersed in a pharmaceutical carrier. Dosage units of the active ingredient are selected from the range of about 0.01 to about 10 mg/kg, preferably about 0.1 to about 1 mg/kg. The dosage units are administered to the human or animal patient from about 1 to about 5 times daily.

Pharmaceutical compositions which contain an active antagonist ingredient of Formula I, comprise a dosage unit which is dissolved or suspended in a standard liquid carrier, such as isotonic saline, and is contained in an ampoule or multiple dose vial suitable for a parenteral injection such as for intravenous, subcutaneous or intramuscular administration. A composition for insufflation may be similar but is usually administered in a metered dose applicator or inhaler. Pulverized powder compositions may, also, be used along with oily preparations, gels. buffers for isotonic preparations, buccal losenges, trans-dermal patches and emulsions or aerosols.

For compounds of this invention, antagonist activity toward the natural antidiuretic hormone (anti-ADH activity) is demonstrated in vivo, in the hydropenic rat and, in vitro, in the medullary tissue of hog kidney. The hydropenic rat screen and the hog kidney assay are known to the art, Huffman et al., U.S. Patent No. 4,469,679.

TABLE I

Pmp-D-Tyr(Et)-Phe-Val-Asn-(D-Cys)-(Tail)

S——————————————S

| Compound (Tail) | Rat in vivo $ED_{300}$ (μg/kg) | Pig in vitro $K_{bind}$ (nM) |
|---|---|---|
| $-Pro-Arg-NH_2$ | 74 | 200 |
| $-D-Arg-Arg-NH_2$ | 25 | 190 |
| $-D-Arg-D-Arg-NH_2$ | 16 | 42 |
| $-N-MeArg-Arg-NH_2$ | 9 | 48 |
| $-Arg-Arg-NH_2$ | 18 | <30 |
| $-Arg-D-Arg-NH_2$ | 19 | <30 |
| $-Arg-NH_2$ | >500 | ca. 30 |

The compounds are tested for partial agonist activity by the following procedures:

PARTIAL AGONIST SCREEN

Adult female mongrel dogs are fasted for 18 hours prior to testing. The studies are conducted under steady state water diuresis initiated by a 5% body weight water load and a 3% dextrose infusion. At 0 hour, indomethacin, a cyclooxygenase inhibitor, is administered (2 mg/kg i.v. bolus and 3 mg/kg/hr i.v. infusion). Twenty minutes later, the test compound is administered i.v. Urine volume and osmolality are measured every 10 minutes for 4 hours.

TABLE II

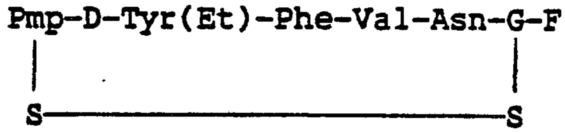

Pmp-D-Tyr(Et)-Phe-Val-Asn-G-F

S—S

| Compound G | F | $U_{osm}$ (Mosm/kg) | Volume (ml/min) | No. of animals |
|---|---|---|---|---|
| D-Cys | Pro-Arg-$NH_2$ | 103 ± 23 | 1.97 ± 0.63 | (3) |
| L-Cys | Pro-Arg-$NH_2$ | 1606 ± 167 | 0.07 ± 0.01 | (6) |
| D-Cys | Arq-D-Arq-$NH_2$ | 112 | 1.6 | (1) |
| L-Cys | Arg-D-Arg-$NH_2$ | 429 ± 221 | 0.7 ± 0.2 | (6) |
| D-Cys | Arg-$NH_2$ | 81 | 2.25 | (1) |
| L-Cys | Arg-$NH_2$ | 946 ± 370 | 1.1 ± 0.7 | (3) |
| Indomethacin | | | | |
| (Control) | | 110 ± 33 | 2.5 ± 0.6 | (6) |
| AVP (3 ng/kg) | | 939 ± 76 | 1.4 ± 0.3 | (3) |
| Without | | | | |
| Indomethacin: | | | | |
| L-Cys | Pro-Arg-$NH_2$ | 294 ± 106 | 0.96 ± 0.2 | (5) |
| AVP (3 ng/kg) | | 599 ± 117 | 0.47 ± 0.13 | (5) |

AVP = arginine vasopressin

TABLE II

The table of biological test results presented above demonstrates that treatment with indomethacin potentiates the antidiuretic activity (i.e. increases urine osmolality and decreases volume) of ADH and potentiates the partial agonist activity of the L-Cys compound such that it subsequently behaves as an agonist. This finding is critical since, in man, the L-Cys compound demonstrated only agonist activity (Dubb et al., Kidney Int. 31:267, January 1987), a property which was not observed in all animal models previously tested. The indomethacin dog model may predict the agonist activity of a compound in man. The finding that the D-Cys isomers displayed greatly diminished agonist activity in this sensitive bioassay system indicates the unexpected advantageous properties of the D-Cys compounds as compared to the L-Cys compounds.

The following examples are not limiting but are illustrative of the invention. All temperatures are in degrees Centigrade.

EXAMPLE I

The vasopressin antagonist compound [1-(β-mercapto-β,β-cyclopentamethylenepropionic acid-2-(0-ethyl)-D-tyrosine-4-valine-6-D-cysteine-7-D-arginine-9-desglycine]-vasopressin is represented by the following structural formula:

```
Pmp-D-Tyr(Et)-Phe-Val-Asn-(D-Cys)-D-Arg-Arg-NH2
 |                           |
 S---------------------------S
```

The protected peptide-resin intermediate, Pmp(MeBzl)-D-Tyr(Et)-Phe-Val-Asn-D-Cys(MeBzl)-D-Arg-(Tos)-Arg (Tos)-BHA was synthesized by solid-phase methods on the automated synthesizer Beckman 990B using benzhydrylamine resin (approx. 0.5 mmol). All amino acids were protected as t-butoxycarbonyl on the amino group and are activated by DCC/HBT for sequential coupling. The Pmp(MeBzl) was coupled using DCC/DMAP. The peptide was cleaved from the resin with removal of the protecting groups by treatment with anhydrous liquid HF (20 mL) in the presence of anisole (2 mL) at 0° for one hour. After removal of the HF under vacuum at 0° the resin was washed with ether. The resin was then extracted with TFA (10 mL), DMF (3 x 10 mL) and glacial HOAc (3 x 10 mL) in such a manner that the extracts were immediately diluted into 1.5 L of water. The pH of the aqueous extracts was adjusted to 7.2 with conc. $NH_4OH$ and the solution was titrated with 0.01 M $K_3Fe(CN)_6$ until a faint yellow color persisted (40 mL, 80% of theory). The pH was then adjusted to 4.5 with glacial HOAc and the solution filtered and then passed over a SM-2 column (5 x 18 cm). The column was washed with water (500 mL) and then eluted with 70% $CH_3CN$/30% water/0.1% TFA. The eluate was evaporated and the residue was dissolved in glacial HOAc, diluted with water and lyophilized, yielding 230 mg crude cyclic peptide.

The crude peptide was dissolved in 0.2 M HOAc and purified by gel filtration on Sephadex® G-15. The appropriate fractions were pooled and lyophilized, yielding 50.8 mg purified peptide, homogenous by hplc (Altex Ultrasphere ODS column, gradient elution 80% A (0.1% TFA), 20% B (0.1% TFA in $CH_3CN$) to 50% A, 50% B over 30 min., flow rate 1.5 mL/min, $k' = 11.03$), FAB mass spec m/z 1138 $(M+H)^+$.

## EXAMPLE 2

The vasopressin antagonist compound [1-(β-mercapto-β,β-cyclopentamethylenepropionic acid-2-(O-ethyl)-D-tyrosine-4-valine-6-D-cysteine-7-D-arginine-8-D-arginine-9-desglycine]-vasopressin is represented by the following structural formula:

```
Pmp-D-Tyr(Et)-Phe-Val-Asn-(D-Cys)-D-Arg-Arg-NH2
 |                           |
 S---------------------------S
```

The protected linear peptide Pmp(MeBzl)-D-Tyr(Et)-Phe-Val-Asn-D-Cys(MeBzl)-D-Arg(Tos)-D-Arg(Tos)-BHA was prepared on a 0.5 mmol scale by the procedure described in Example 1 substituting D-Arg(Tos) for Arg(Tos) in the 8-position. The peptide was cleaved from the resin with removal of the protecting groups by treatment with anhydrous liquid HF (20 mL) in the presence of anisole (2 mL) at 0° for one hour. After removal of the HF under vacuum at 0° the resin was washed with ether. The resin was then extracted with TFA (10 mL), DMF (3 x 10 mL) and glacial HOAc (3 x 10 mL) in such a manner that the extracts were immediately diluted into 1.5 L of water. The pH of the aqueous extracts was adjusted to 7.2 with conc. $NH_4OH$ and the solution was titrated with 0.01 M $K_3Fe(CN)_6$ until a faint yellow color persisted (35 mL, 70% of theory). The pH was then adjusted to 4.5 with glacial HOAc and the solution filtered and then passed over a SM-2 column (5 x 18 cm). The column was washed with water (500 mL) and then eluted with 60% $CH_3CN$/40% water/0.1% TFA. The eluate was evaporated and the residue was dissolved in glacial HOAc, diluted with water and lyophilized, yielding 294 mg crude cyclic peptide.

The crude peptide was purified by counter current distribution using the solvent system n-BuOH/HOAc/water 4:1:5. The appropriate fractions were pooled and lyophilized, yielding 96 mg partially

purified peptide. The peptide was further purified by gel filtration on Sephadex® G-15 using 0.2 M HOAc as eluant. The appropriate fractions were pooled and lyophilized, yielding 30 mg purified peptide, homogenous by hplc (Altex Ultrasphere ODS column, gradient elution 80% A (0.1% TFA), 20% B (0.1% TFA in $CH_3CN$) to 50% A, 50% B over 30 min., flow rate 1.5 mL/min, $k' = 11.5$), FAB mass spec m/z 1138 $(M+H)^+$.

EXAMPLE 3

The vasopressin antagonist compound [1-(β-mercapto-β,β-cyclopentamethylenepropionic acid-2-(O-ethyl)-D-tyrosine-4-valine-6-D-cysteine-7-N-methylarginine-9-desglycine]-vasopressin is represented by the following structural formula.

4'-MePmp-D-Try(Et)-Phe-Val-Asn-(D-Cys)-N-MeArg-Arg-$NH_2$

S————————————S

The protected peptide-resin intermediate, Pmp(MeBzl)-D-Tyr(Et)-Phe-Val-Asn-D-Cys(MeBzl)-MeArg-(Tos)-Arg(Tos)-BHA, is synthesized by solid-phase methods on the automated synthesizer Beckman 990B using benzhydrylamine resin (0.5 mmol) by the procedure of Example 1.

The peptide was cleaved from the resin with removal of the protecting groups by treatment with anhydrous liquid HF (20 mL) in the presence of anisole (2 mL) at 0° for one hour. After removal of the HF under vacuum at 0° the resin was washed with ether. The resin was then extracted with TFA (10 mL), DMF (3 x 10 mL) and glacial HOAc (3 x 10 mL) in such a manner that the extracts were immediately diluted into 1.5 L of water. The pH of the aqueous extracts was adjusted to 7.2 with conc. $NH_4OH$ and the solution was titrated with 0.01 M $K_3Fe(CN)_6$ until a faint yellow color persisted (35 mL, 70% of theory). The pH was then adjusted to 4.5 with glacial HOAc and the solution filtered and then passed over a SM-2 column (5 x 18 cm). The column was washed with water (500 mL) and then eluted with 60% $CH_3CN$/40% water/0.1% TFA. The eluate was evaporated and the residue was dissolved in glacial HOAc, diluted with water and lyophilized, yielding 137 mg crude cyclic peptide.

The crude peptide was purified by counter current distribution using the solvent system n-BuOH/HOAc/water 4:1:5. The appropriate fractions were pooled and lyophilized, yielding 60 mg partially purified peptide.

The peptide was further purified by preparative hplc using 40% $CH_3CN$, 60% water, 0.1% TFA as eluant and an Altex Ultrasphere® ODS column (9.4 mm x 30 cm). The appropriate fractions were pooled, evaporated to dryness, dissolved in 1% HOAc and lyophilized, yielding purified peptide, homogeneous by hplc (Altex Ultrasphere® ODS column, gradient elution 80% A (0.1% TFA), 20% B (0.1% TFA in $CH_3CN$) to 50% A, 50% B over 30 min., flow rate 1.5 mL/min, $k' = 11.46$), FAB mass spec m/z 1152 $(M+H)^+$.

EXAMPLE 4

The vasopressin antagonist compound [1-(β-mercapto-β,β-cyclopentamethylenepropionic acid-2-(O-ethyl)-D-tyrosine-4-valine-6-D-cysteine-7-arginine-9-desglycine]-vasopressin is represented by the following structural formula:

Pmp-D-Tyr(Et)-Phe-Val-Asn-(D-Cys)-D-Arg-Arg-$NH_2$

S————————————S

The protected linear peptide, Pmp(MeBzl)-D-Tyr(Et)-Phe-Val-Asn-D-Cys(MeBzl)-Arg(Tos)- Arg(Tos)-BHA was prepared by procedure described in Example 1 on a 0.5 mmol scale. The peptide was cleaved from the resin with removal of the protecting groups by treatment with anhydrous liquid HF (20 mL) in the presence of anisole (2 mL) at 0° for one hour. After removal of the HF under vacuum at 0° the resin was

washed with ether. The resin was then extracted with TFA (10 mL), DMF (3 x 10 mL) and glacial HOAc (3 x 10 mL) in such a manner that the extracts were immediately diluted into 1.5 L of water. The pH of the aqueous extracts was adjusted to 7.2 with conc. $NH_4OH$ and the solution was titrated with 0.01 M $K_3Fe(CN)_6$ until a faint yellow color persisted (40 mL, 80% of theory). The pH was then adjusted to a 4.5 with glacial HOAc and the solution filtered and then passed over a SM-2 column (5 x 18 cm). The column was washed with water (500 mL) and then eluted with 60% $CH_3CN$/40% water/0.1% TFA. The eluate was evaporated and the residue was dissolved in glacial HOAc, diluted with water and lyophilized, yielding 185 mg crude cyclic peptide.

The crude peptide was purified by counter current distribution using the solvent system n-BuOH/HOAc/water 4:1:5. The appropriate fractions were pooled and lyophilized, yielding 95 mg partially purified peptide. The peptide was further purified by gel filtration on Sephadex® G-15 using 0.2 M HOAc as eluant. The appropriate fractions were pooled and lyophilized, yielding 50 mg purified peptide, homogeneous by hplc (Altex Ultrasphere® ODS column, gradient elution 80% A (0.1% TFA), 20% B (0.1% TFA in $CH_3CN$) to 50% A, 50% B over 30 min., flow rate 1.5 mL/min, $k' = 13.76$), FAB mass spec m/z 1138 $(M+H)^+$.

## EXAMPLE 5

The vasopressin antagonist compound [1-(β-mercapto-β,β-cyclopentamethylenepropionic acid-2-(O-ethyl)-D-tyrosine-4-valine-6-D-cysteine-7-arginine-8-D-arginine-9-desglycine]-vasopressin is represented by the following structural formula:

Pmp-D-Try(Et)-Phe-Val-Asn-(D-Cys)-D-Arg-Arg-$NH_2$

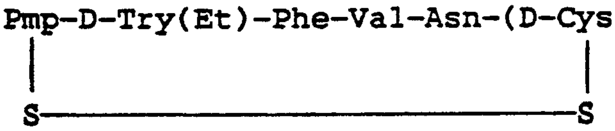

The protected linear peptide, Pmp(MeBzl)-D-Tyr(Et)-Phe-Val-Asn-D-Cys(MeBzl)-Arg(Tos)-D-Arg(Tos)-BHA was prepared by procedure described in Example 1 on a 0.5 mmol scale. The peptide was cleaved from the resin with removal of the protecting groups by treatment with anhydrous liquid HF (20 mL) in the presence of anisole (2 mL) at 0° for one hour. After removal of the HF under vacuum at 0° the resin was washed with ether. The resin was then extracted with TFA (10 mL), DMF (3 x 10 mL) and glacial HOAc (3 x 10 mL) in such a manner that the extracts were immediately diluted into the 1.5 L of water. The pH of the aqueous extracts was adjusted to 7.2 with conc. $NH_4OH$ and the solution was titrated with 0.01 M $K_3Fe(CN)_6$ until a faint yellow color persisted (40 mL, 80% of theory). The pH was then adjusted to 4.5 with glacial HOAc and the solution filtered and then passed over a SM-2 column (5 x 18 cm). The column was washed with water (500 mL) and then eluted with 60% $CH_3CN$/40% water/0.1% TFA. The eluate was evaporated and the residue was dissolved in glacial HOAc, diluted with water and lyophilized, yielding 210 mg crude cyclic peptide.

The crude peptide was purified by counter current distribution using the solvent system n-BuOH/HOAc/water 4:1:5. The appropriate fractions were pooled and lyophilized, yielding 100 mg partially purified peptide. The peptide was further purified by gel filtration on Sephadex® G-15 using 0.2 M HOAc as eluant. The appropriate fractions were pooled and lyophilized, yielding 54 mg purified peptide, homogeneous by hplc (Altex Ultrasphere® ODS column, gradient elution 80% A (0.1% TFA), 20% B (0.1% TFA in $CH_3CN$) to 50% A, 50% B over 30 min., flow rate 1.5 mL/min, $k' = 13.76$), FAB mass spec m/z 1138 $(M+H)^+$.

## EXAMPLE 6

The vasopressin antagonist compound [1-(β-mercapto-β,β-cyclopentamethylenepropionic acid-2 (O-ethyl)-D-tyrosine-4-valine-6-D-cysteine-7-desproline-9-desglycine]-vasopressin is represented by the following structural formula:

Pmp-D-Tyr(Et)-Phe-Val-Asn-(D-Cys)-Arg-$NH_2$

S——————————————S

The protected linear peptide, Pmp(MeBzl)-D-Tyr(Et)-Phe-Val-Asn-D-Cys(MeBzl)-D-Arg(Tos)-Arg (Tos)-BHA was prepared by solid-phase methods, as described hereabove, on a 0.5 mmol scale. The peptide was cleaved from the resin with removal of the protecting groups by treatment with anhydrous liquid HF (20 mL) in the presence of anisole (2 mL) at 0° for one hour. After removal of the HF under vacuum at 0° the resin was washed with ether. The resin was then extracted with TFA (10 mL), DMF (3 x 10 mL) and glacial HOAc (3 x 10 mL) in such a manner that the extracts were immediately diluted into 1.5 L of water. The pH of the aqueous extracts was adjusted to 7.2 with conc. $NH_4OH$ and the solution was titrated with 0.01 M $K_3Fe(CN)_6$ until a faint yellow color persisted (40 mL, 80% of theory). The pH was then adjusted to 4.5 with glacial HOAc and the solution filtered and then passed over a SM-2 column (5 x 18 cm). The column was washed with water (500 mL) and then eluted with 60% $CH_3CN$/40% water/0.1% TFA. The eluate was evaporated and the residue was dissolved in glacial HOAc, diluted with water and lyophilized, yielding 120 mg crude cyclic peptide.

The crude peptide was purified by counter current distribution using the solvent system n-BuOH/HOAc/water 4:1:5. The appropriate fractions were pooled and lyophilized, yielding 40 mg partially purified peptide. The peptide was further purified by gel filtration on Sephadex® G-15 using 0.2 M HOAc as eluant. The appropriate fractions were pooled and lyophilized, yielding 21 mg purified peptide, homogenous by hplc (Altex Ultrasphere® ODS column, gradient elution 80% A (0.1% TFA), 20% B (0.1% TFA in $CH_3CN$) to 50% A, 50% B over 30 min., flow rate 1.5 mL/min, $k' = 12.67$), FAB mass spec m/z 982 $(M+H)^+$.

## EXAMPLE 7

The vasopressin antagonist compound [1-(β-mercapto-β,β-cyclopentamethylenepropionic acid-2-(O-ethyl)-D-tyrosine-4-valine-6-D-cysteine- 9-desglycine]-vasopressin is represented by the following structural formula:

Pmp-D-Try(Et)-Phe-Val-Asn-(D-Cys)-Pro-Arg-$NH_2$

S——————————————S

The protected linear peptide Pmp(MeBzl)-D-Tyr(Et)-Phe-Val-Asn-D-Cys(MeBzl)-Pro-Arg(Tos)- ArgBHA was prepared by solid-phase methods as described in Example 1, on a 2.0 mmol scale. After HF cleavage at 0° in the presence of anisole, extraction from the resin and oxidation with 0.01 M $K_3Fe(CN)_6$ in the usual manner, the peptide was absorbed onto a 3 inch by 28 inch $C_{18}$ reverse phase open column. The peptide was eluted with a linear gradient of 30% $CH_3CN$/0.1% TFA (1 L) to 60% $CH_3CN$/0.1% TFA (1 L). The appropriate fractions were pooled, evaporated to dryness and lyophilized from 0.2 M HOAc to yield 528 mg purified peptide, homogenous by hplc (Altex Ultrasphere® ODS column, gradient elution 80% A (0.1% TFA), 20% B (0.1% TFA in $CH_3CN$) to 50% A, 50% B over 20 min., flow rate 1.5 mL/min, $k' = 4.68$), FAB mass spec m/z 1079 $(M+H)^+$, amino acid analysis: Arg, 1.04; Asp, 0.97; Pro, 0.98; Cys, 0.33; Val, 0.97; Tyr, 0.82; Phe, 1.16.

## EXAMPLE 8

The vasopressin antagonist compound [1-(β-mercapto-β,β-cyclopentamethylenepropionic acid 2-(O-ethyl)-D-tyrosine-4-valine-6-D-cysteine-7-arginine-8-glycine-9-glycine-10-arginine]-vasopressin is represented by the following structural formula:

Pmp-D-Tyr(Et)-Phe-Val-Asn-(D-Cys)-Arg-Gly-Gly-Arg-$NH_2$

S————————————————S

The protected peptide-resin intermediate, Pmp(MeBzl)-D-Tyr(Et)-Phe-Val-Asn-D-Cys(MeBzl)-Arg(Tos)-Gly-Gly-Arg(Tos)-BHA is synthesized by solid-phase methods on the automated synthesizer Beckman 990B using 1.0 g of benzhydrylamine resin. All amino acids are protected as t-butoxycarbonyl on the nitrogen and are activated by DCC/HBT for sequential coupling. The Pmp(MeBzl) is coupled using DMAP/DCC. The peptide is cleaved from the resin with simultaneous deprotection of the side-chain protecting groups using anhydrous liquid HF (30 mL) in the presence of anisole (3.0 ml) at 0° for one hour. After evaporation in vacuo to dryness, the residue containing peptide is washed with anhydrous ether. The crude peptide is extracted with dimethylformamide (100 ml) and 40% acetic acid (100 ml) and the extracts are added to 3.5 liters of degassed water. The aqueous diluted disulfhydryl decapeptide mixture is oxidatively cyclized with 110 ml of potassium ferricyanide (0.01 M) at a pH of 7.2. The pH of the solution is adjusted to 4.5 using glacial acetic acid. The solution is passed through a weakly acid acrylic resin (Bio-Rex 70) column. The column is eluted with pyridine-acetate buffer (30:4:66, pyridine/glacial acetic acid/water v/v). The pyridine acetate is removed by distillation in vacuo. The residue is lyophilized from dilute acetic acid to give the product of partially purified crude peptide.

## EXAMPLE 9

Preparation of:

Pmp-D-Tyr(Et)-Phe-Val-Asn-(D-Cys)-Pro(OH)

S————————————————S

Boc-Pro-Merrifield resin is made by coupling Boc-Pro to Merrifield resin using the cesium salt method to give Boc-Pro-$OCH_2C_6H_4$-resin which is used as the starting material for the synthesis. The synthesis is carried out on the Beckman 990 B peptide synthesizer using the following protocol. Three equivalents of the amino acids are dissolved in their appropriate solvents [the Boc derivatives of D-Cys(MeBzl), Val, Phe and Pmp(MeBzl) in methylene chloride, Asn in dimethylformamide, X such as D-Tyr(Et) or BrZ-D-Tyr in 1:1 methylene chloride/dimethylformamide) and are coupled using an equimolar amount of dicyclohexylcarbodiimide (DCC) and 1-hydroxybenzotriazole (HBT) except for the coupling of Pmp-(MeBzl) where 1.0 equivalent of dimethylaminopyridine is used as catalyst. The extent of coupling is determined by qualitative ninhydrin analyses of each aliquot sample and couplings are repeated when necessary. The Boc groups are removed using 1:1 trifluoroacetic acid/methylene chloride and, after washing, the free amine is generated using 5% diisopropylethylamine/methylene chloride. The sequence of the peptide is checked using solid phase sequencing before the coupling of the Pmp(MeBzl) and its homogeneity confirmed.

1.1 Grams (0.5 mmole) of the protected heptapeptide resin with 3 ml of anisole is stirred 60 min at 0° (ice bath) in 25 ml of anhydrous liquid hydrogen fluoride (HF). The HF is then removed under reduced pressure at 0°. The residue is washed with ethyl ether (4 x 20 ml, discarded) and the peptide extracted with dimethylformamide (3 x 10 ml), 20% acetic acid (3 x 10 ml) and 0.3N ammonium hydroxide (3 x 10 ml).

The extracts are added to 2 l of degassed water and pH adjusted to 7.1 with conc. ammonium hydroxide. A 0.01M solution of potassium ferricyanide is then added dropwise with stirring until a faint yellow color persisted. The solution is adjusted to pH 4.5.

The resulting solution is then passed through a flash column (5 cm x 15 cm) of a packing of silica gel coated with a C-18 silane. The column is then washed with 350 ml of water and the peptide eluted with 500 ml of 1:1 acetonitrile/water (0.25% trifluoroacetic acid in 20 ml fractions.

The appropriate fractions are combined and concentrated. The residue is dissolved in conc. acetic acid diluted with water and lyophilized to give the title peptide which is used without further purification for the synthesis of the tail modified peptides.

## EXAMPLE 10

Preparation of:

$$Pmp\text{-}D\text{-}Tyr(Et)\text{-}Phe\text{-}Val\text{-}Asn\text{-}(D\text{-}Cys)\text{-}Pro\text{-}NH(CH_2)_5\text{-}NH_2$$

S————————————————S

1,5-Diaminopentane (14.0 ml, 120 mmol) is dissolved in tert-butanol(70 ml) and is treated dropwise over a period of 10 min with di-tert-butyl dicarbonate (9.2 ml, 40 mmol). After the addition is completed, the reaction mixture is stirred at room temperature for 16.5 hr. The reaction is then treated with 1N sodium hydroxide solution (aq) (90 ml), stirred for 1 hr and finally extracted with chloroform. The chloroform extracts are dried ($MgSO_4$) and concentrated under vacuum. The residue is dissolved in water, made acidic (pH = 2) by the dropwise addition of 3N hydrochloric acid at 0° and washed with ether to remove the diprotected diamine. The aqueous portion is made basic (pH 10) with 5% sodium carbonate solution and was extracted with ethyl acetate to give mono-Boc 1,5-diaminopentane. The structure is confirmed by 'H NMR and CI-MS.

To a solution of the heptapeptide prepared in Example 9 and mono-Boc-1.5-diaminopentane in dimethylformamide, dicyclohexylcarbodiimide and 1-hydroxybenzotriazole hydrate is added. The reaction mixture is stirred at room temperature for 19 hours. The dimethylformamide is then removed under vacuum. The residue is treated with trifluoroacetic acid at 0° for 2 hours. After this time, the trifluoroacetic acid is removed under vacuum and the residue in 1% acetic acid is passed over a Bio-Rex® 70 ($H^+$) ion exchange column. The basic products are washed off the ion exchange column with pyridine buffer ($H_2O$/pyridine/HOAc, 66:30:4) and evaporated to give crude product.

## EXAMPLE 11

Preparation of:

$$Pmp\text{-}D\text{-}Tyr(Et)\text{-}Phe\text{-}Val\text{-}Asn\text{-}(D\text{-}Cys)\text{-}Pro\text{-}NH\text{-}(CH_2)_4NHC(=NH)NH_2$$

S————————————————S

Mono-Boc-1,4-diaminobutane (1.25 g, 6.6 mmol), prepared from putrescine, in dioxane (2 ml) and water (6.5 ml) was treated with O-methylisourea hydrogen sulfate (1.25 g, 7.26 mmol) and 2N sodium hydroxide (aq) (3.75 ml) at room temperature. The resulting solution was stirred for 6 days. The solvent was removed under reduced pressure and the residue made basic (pH = 12) by the addition of 2N sodium hydroxide. The residue was again evaporated, taken up in ethyl acetate, filtered and evaporated. The crude guanidine was dried by evaporation from toluene and used without further purification.

The crude guanidine (410 mg, 1.78 mmol) in 2N sodium hydroxide (aq) (2 ml) and water (2 ml) was treated at room temperature with p-toluenesulfonyl chloride (340 mg, 1.78 mmol) for 18 hours. The pH was adjusted to 8 with 5% sodium carbonate solution. The mixture was extracted with ethyl acetate to give, upon evaporation, 437 mg of crude product. Purification by flash chromatography (3 x 15 cm silica bed, 80% ethyl acetate/hexane) gave 265 mg (39%) of the desired tosylated product whose identity was confirmed by ′H NMR and CI-mass spectroscopy.

The Tos-NHC(=NH)NH($CH_2$)$_4$-NHBoc (108 mg, 0.281 mmol) in methylene chloride (1 ml) was treated with trifluoroacetic acid (1 ml) at 0° for 40 minutes. The reaction was evaporated under vacuum, the residue adjusted to pH 8 with 5% sodium carbonate solution and evaporated to dryness. The residue was taken up into ethyl acetate, filtered and evaporated. The crude des-Boc product was dried by evaporation from toluene to give 66 mg (82%). Identity was confirmed by H NMR and used without further purification.

[1-(β-mercapto-β,β-cyclopentamethylene-propionic acid)-2-D-(O-ethyl)tyrosine-4-valine-6-D-cysteine-8-desarginine-9-desglycinamide]-vasopressin, prepared as in Example 9, in dimethylformamide is treated at room temperature with the tosylguanidinobutylamine. The mixture is stirred for 43 hours. The solvent is

removed at reduced pressure and the residue is dissolved in trifluoroacetic acid, then treated at room temperature with trifluoromethanesulfonic acid and anisole with stirring for 2 hours. The reaction mixure is evaporated to dryness, dissolved in 10% acetic acid, filtered and passed through a Bio-Rex® 70 column. The crude guanidine is eluted off the column with pyridine buffer (pyridine/water/acetic acid, 30:66:4), evaporated to afford the crude peptide.

## EXAMPLE 12

Substituting a stoichiometric quantity of Boc-L-Tyr(Et) for Boc-D-Tyr(Et) at the second unit of the peptide synthesis of Example 7 gives cyclized

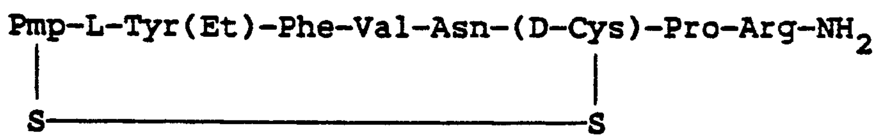

Substituting in Example 7, Boc-D Ile for Boc-D-Tyr(Et) at the second unit gives

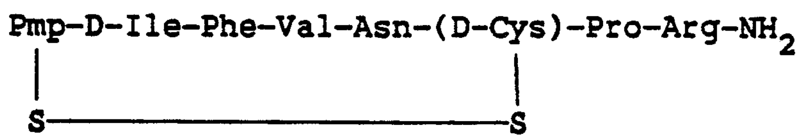

Substituting Boc-L-Phe(4-Me) for the amino acid at the third unit and Boc-Nle at the fourth unit in the synthesizer sequence reactions of Example 7 gives:

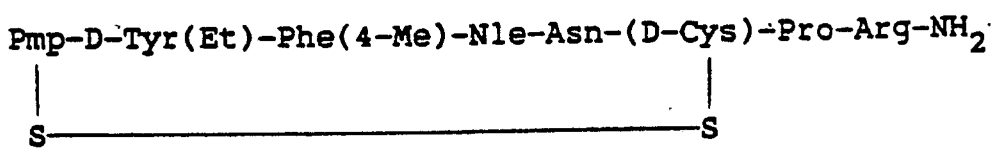

Substituting Boc-Cha at the fourth unit gives

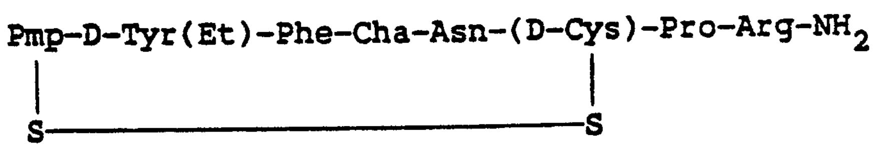

Substituting unprotected Gln at the fourth unit using HBT gives

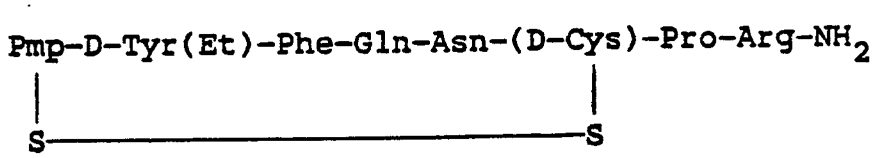

## EXAMPLE 13

Substituting the appropriate protected ring units in the snythetic sequence of Example 1 and using the appropriate acid gives the respective peptide salt as follows:

[1-(β-mercapto-β,β-cyclopentamethylene propionic acid)-2-tyrosine-3-(4′-methylphenylalanine)-6-D-cysteine-7-D-arginine-8-arginine-9-desglycine]-vasopressin acetate;
[1-(β-mercapto-β,β-cyclopentamethylene propionic acid)-2-D-phenylalanine-4-isoleucine-6-D-cysteine-7-D-arginine-8-arginine-9-desglycine]-vasopressin phosphate;
[1-(β-mercapto-β,β-cyclopentamethylene propionic acid)-2-D-isoleucine-4-cyclohexylglycine-6-D-cysteine-7-D-arginine-8-arginine-9-desglycine]-vasopressin hydrochloride.

## EXAMPLE 14

The vasopressin antagonist compound [1-(β-mercapto-β,β-cyclopentamethylenepropionic acid)-2-(O-ethyl)-D-tyrosine-4-valine-6-D-cysteine-7-ornithine-8-ornithine-9-ornithine]-vasopressin is represented by the following structural formula:

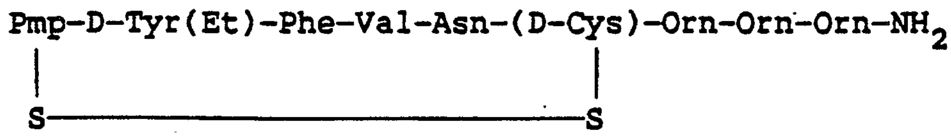

The protected peptide-resin intermediate, Pmp(MeBzl)-D-Tyr(Et)-Phe-Val-Asn-D-Cys(MeBzl)-Orn(ClZ)-Orn(ClZ)-Orn(ClZ)-BHA is synthesized by solid-phase methods on the automated synthesizer Beckman 990B using 1.0 g of benzyhydrylamine resin. All amino acids are protected as t-butyloxycarbonyl on the nitrogen and are activated by DCC/HBT for sequential coupling. The Pmp(MeBzl) is coupled using DMAP/DCC. The peptide is cleaved from the resin with simultaneous deprotection of the side-chain protecting groups using anhydrous HF (30 ml) in the presence of anisole (3.0 ml) at 0° C for 60 minutes. After evaporation in vacuo to dryness, the residue containing peptide is washed with anhydrous ether. The crude peptide is extracted with dimethylformamide (100 ml) and 40% acetic acid (100 ml) and the extracts are added to 3.5 liters of degassed water. The aqueous diluted disulfhydryl nonapeptide mixture is oxidatively cyclized with 110 ml of potassium ferricyanide (0101 M) at a pH of 7.2. The pH of the solution is adjusted to 4.5 using glacial acetic acid (HOAc). The solution is passed through a weakly acid acrylic resin (Bio Rex® 70) column. The column is eluted with pyridine acetate-buffer (30:4:66, pyridine/glacial acetic acid/water v/v). The pyridine acetate is removed by distillation in vacuo. The residue is lyophilized from dilute acetic acid to give the product of partially purified crude peptide.

## EXAMPLE 15

The vasopressin antagonist compound [1-(β-mercapto-β,β-cyclopentamethylenepropionic acid)-2-(O-ethyl)-D-tyrosine-4-valine-6-D-cysteine-7-lysine-8-lysine-9-lysine]-vasopressin is represented by the following structural formula:

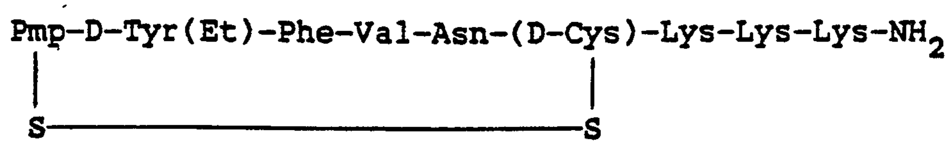

The protected peptide-resin intermediate, Pmp(MeBzl)-D-Tyr(et)-Phe-Val-Asn-D-Cys(MeBzl)-Lys(ClZ)-Lys(ClZ)-Lys(ClZ)-BHA is synthesized by solid-phase methods on the automated synthesizer Beckman 990B using 1.0 g of benzyhydrylamine resin. All amino acids are protected as t-butyloxycarbonyl on the nitrogen and are activated by DCC/HBT for sequential coupling. The MePmp(MeBzl) is coupled using DMAP/DCC. The peptide is cleaved from the resin with simultaneous deprotection of the side-chain protecting groups using anhydrous HF (30 ml) in the presence of anisole (3.0 ml) at 0° for 60 minutes. After evaporation in vacuo to dryness, the residue containing peptide is washed with anhydrous ether. The crude peptide is extracted with dimethylformamide (100 ml) and 40% acetic acid (100 ml) and the extracts are added to 3.5 liters of degassed water. The aqueous diluted disulfhydryl nonapeptide mixture is oxidatively cyclized with 110 ml of potassium ferricyanide (0.01 M) at a pH of 7.2. The pH of the solution is

adjusted to 4.5 using glacial acetic acid (HOAc). The solution is passed through a weakly acid resin (Bio-Rex® 70) column. The column is eluted with pyridine-acetate buffer (30:4:66, pyridine/glacial acetic acid/water v/v). The pyridine acetate is removed by distillation in vacuo. The residue is lyophilized from dilute acetic acid to give the product of partially purified crude peptide.

## EXAMPLE 16

The vasopressin antagonist compound [1-(β-mercapto-β,β-cyclopentamethylenepropionic acid)-2-(O-ethyl)-D-tyrosine-4-valine-6-D-cysteine-8-arginine-9-arginine]-vasopressin is represented by the following structural formula:

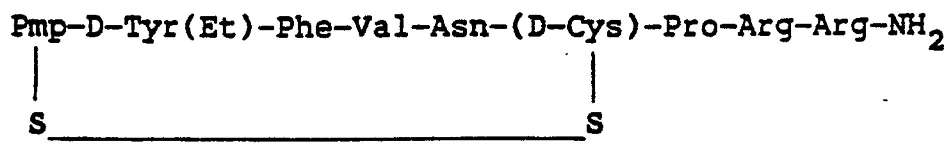

The protected peptide-resin intermediate, Pmp(MeBzl)-D-Tyr(Et)-Phe-Val-Asn-D-Cys(MeBzl)-Pro-Arg-(Tos)-Arg(Tos)-BHA is synthesized by solid-phase methods on the automated synthesizer Beckman 990 B using 1.0 g of benzyhydrylamine resin. All amino acids are protected as t-butyloxycarbonyl on the nitrogen and are activated by DCC/HBT for sequential coupling. The Pmp(MeBzl) is coupled using DMAP/DCC. The peptide is cleaved from the resin with simultaneous deprotection of the side-chain protecting groups using anhydrous HF (30 ml) in the presence of anisole (3.0 ml) at 0 °C for 60 minutes. After evaporation in vacuo to dryness, the residue containing peptide is washed with anhydrous ether. The crude peptide is extracted with dimethylformamide (100 ml) and 40% acetic acid (100 ml) and the extracts are added to 3.5 liters of degassed water. The aqueous diluted disulfhydryl nonapeptide mixture is oxidatively cyclized with 110 ml of potassium ferricyanide (0101 M) at a pH of 7.2. The pH of the solution is adjusted to 4.5 using glacial acetic acid (HOAc). The solution is passed through a weakly acid acrylic resin (Bio-Rex® 70) column. The column is eluted with pyridine-acetate buffer (30:4:66, pyridine/glacial acetic acid/water v/v). The pyridine acetate is removed by distillation in vacuo. The residue is lyophilized from dilute acetic acid to give the product of partially purified crude peptide.

## EXAMPLE 17

Using β-mercapto-β,β-cyclotetramethylenepropionic acid in place of the cyclopentamethylene compound in the procedure of Example 7 gives [1-(β-mercapto-β,β-cyclotetramethylenepropionic acid)-2-(O-ethyl)-D-tyrosine-4-valine-6-D-cysteine-7-D-arginine-9-desglycine]-vasopressin which is represented by the following structural formula:

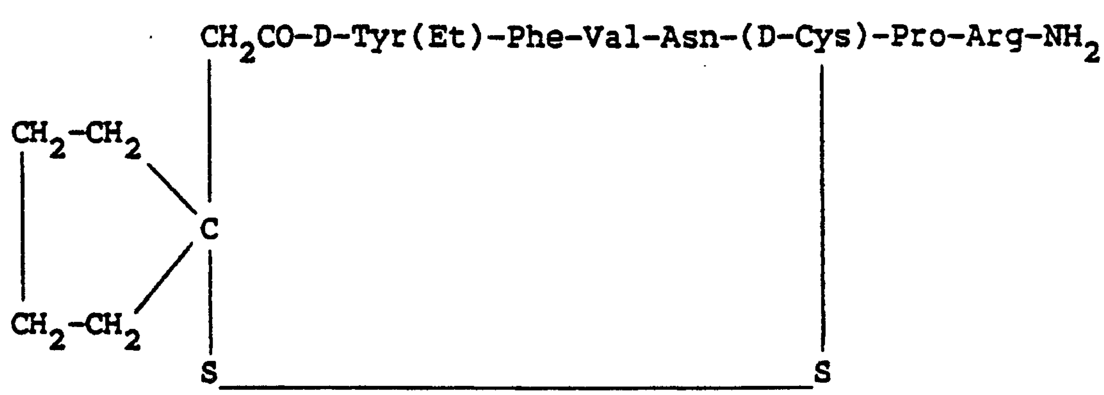

EXAMPLE 18

Parenteral Dosage Unit Compositions:

A preparation which contains 0.10 mg of the peptide of Example 7 as a sterile dry powder for parenteral injection is prepared as follows: 0.5 mg of peptide is dissolved in 1 ml of an aqueous solution of 20 mg of mannitol. The solution is filtered under sterile conditions into a 2 ml ampoule and lyophilized. The reconstituted solution is administered to a patient in need of vasopressin antagonist treatment as necessary, from 1-5 times daily by injection, or in an equivalent continuous i.v. drip injection.

Nasal Dosage Unit Compositions:

2.5 Mg of a finely ground peptide of this invention, such as the product of Example 1, is suspended in a mixture of 75 mg of benzyl alcohol and 1.395 g of a suspending agent such as a commercial mixture of semi-snythetic glycerides of higher fatty acids. The suspension is placed in an aerosol 10 ml container which is closed with a metering valve and charged with aerosol propellants. The contents comprise 100 unit doses which are administered intranasally to a subject in need thereof from 1-6 times a day.

**Claims**

1. A compound of the formula:

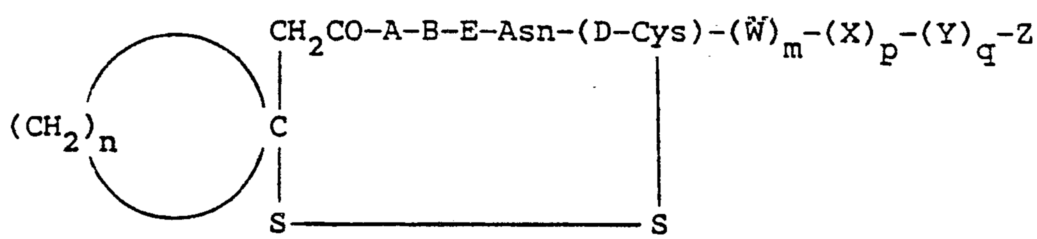

in which:
A is a D or L isomer of Phe, Phe(4′-Alk), Ile, Cha, Tyr, or Tyr(O-Alk);
B is Phe, Phe(4′Alk), Tyr(O-Alk), Ile or Tyr;
E is Val, Ile, Abu, Chg,, Gln, Lys, Cha, Nle, Leu, Ala or Gly;
n is 4 or 5;
m, p and q are each 0 or 1;
W is a D or L isomer of Pro, Arg, HArg, N-MeArg, Lys or Orn;
X is a D or L isomer of Arg, Lys, Orn or Gln; or when m and q are 1, X may be Gly;
Y is a D or L isomer of Arg, Lys, Orn, Ser, Gln, Tyr or Ala;
Z is NH-$(CH_2)_t$-NHR; a D or L isomer of Arg-$NH_2$, Lys-$NH_2$ or Orn-$NH_2$; or, when at least one of m, p and q is 1, NHR′ or OH;
R is H or C(=NH)-$NH_2$;
R′ is H or Alk;
t is 2-6; and
Alk is alkyl having 1-4 carbon atoms;
or a pharmaceutically acceptable acid addition salt or lower alkyl or benzyl ester thereof.

2. A compound of claim 1 in which A is D-Tyr or D-Tyr(Et); B is Phe; E is Val; m and p are 1 and q is 0; W is a D or L isomer of Pro, Arg or N-MeArg; X is a D or L isomer of Arg or Gly; and Z is NHR or a D or L isomer of Arg-$NH_2$.

3. A compound of claim 1 or 2 in which $(W)_m$-$(X)_p$-$(Y)_q$-Z is Pro-Arg-$NH_2$, D-Arg-Arg-$NH_2$, Arg-Arg-$NH_2$, Arg-D-Arg-$NH_2$, D-Arg-D-Arg-$NH_2$, or N-MeArg-Arg-$NH_2$.

4. A compound of claim 1 in which $(W)_m$-$(X)_p$-$(Y)_q$-Z is Pro-NH$(CH_2)_4$NHC(=NH)-$NH_2$, Pro-Arg-NH-$(CH_2)_2NH_2$, Arg-$NH_2$ or Arg-Gly-Arg $NH_2$.

5. A compound according to claim 2 in which $(W)_mO(X)_p$-$(Y)_q$-Z is Pro-Arg-$NH_2$, D-Arg-Arg-$NH_2$ or Arg-D Arg-$NH_2$.

6. A compound according to claim 1 which is: [1 (β-mercapto-β,β-cyclopentamethylenepropionic acid)-2-(O-ethyl)-D-tyrosine-4-valine-6-D-cysteine-7-D-arginine-9-desglycine]vasopressin;
[1-(β-mercapto-β,β-cyclopentamethylenepropionic acid)-2-(O-ethyl)-D-tyrosine-4-valine-6-D-cysteine-9-desglycine]vasopressin; or
[1-(β-mercapto-β,β-cyclopentamethylenepropionic acid)-2-(O-ethyl)-D-tyrosine-4-valine 6-D-cysteine-7-arginine-8-D-arginine-9-desglycine]vasopressin.

7. A compound according to any one of claims 1 to 6 for use as a medicament.

8. A pharmaceutical composition comprising a compound according to any one of claims 1 to 6 and a pharmaceutical carrier.

9. A process for preparing a compound of formula (I) or a pharmaceutically acceptable addition salt as defined in claim 1, which comprises:

a) (i) for compounds in which Z is other than NH $(CH_2)_t$-NHR, cyclising by means of two mercapto groups, an optionally protected compound of Formula II:

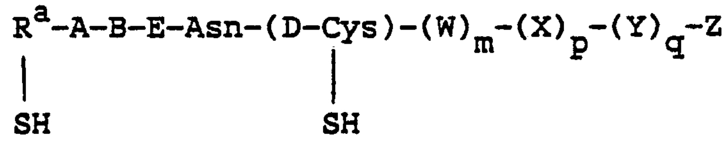

(II)

in which $R^a$ is β-mercapto-β,β-cyclopenta(or cyclotetra)methylenepropionic acid and A, B, E, W, X, Y, Z, m, p and q are as defined in claim 1, with the mercapto groups being members of the $R^a$ and D-Cys units; or

(ii) for compounds in which Z is NH-$(CH_2)_t$-NHR, reacting a compound of the formula:

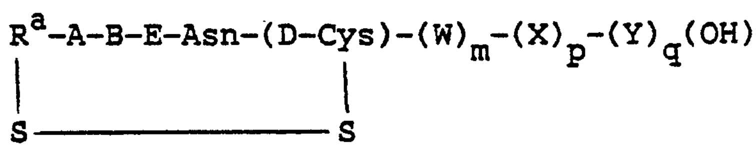

with a compound of the formula: $NH_2$-$(CH_2)_t$-NHR in which $R^a$ is as hereinbefore defined and A, B, E, W, X, Y, m, p, q, t and R are as defined in claim 1;

b) removing any protecting groups, if necessary;

c) optionally forming a lower alkyl or benzyl ester; and

d) optionally forming a pharmaceutically acceptable acid addition salt.

10. An intermediate compound of formula (II):

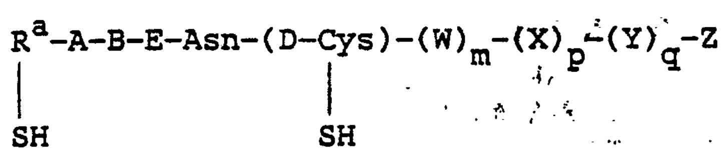

wherein:
$R^a$ is β-mercapto-β,β-cyclopenta(or cyclotetra)methylene-propionic acid and A, B, E, W, X, Y, Z, m, p and q are as defined in claim 1.

Claims for the following Contracting State: (ES)

1. A process for preparing a compound of the formula (I):

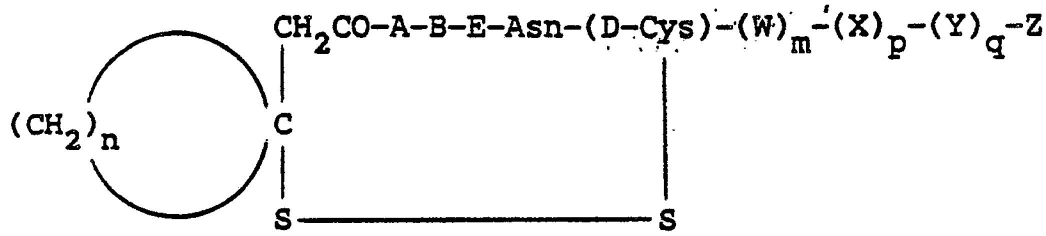

(I)

in which:
A is a D or L isomer of Phe, Phe(4′-Alk), Ile, Cha, Tyr, or Tyr(O-Alk);
B is Phe, Phe(4′-Alk), Tyr(O-Alk), Ile or Tyr;
E is Val, Ile, Abu, Chg,, Gln, Lys, Cha, Nle, Leu, Ala or Gly;
n is 4 or 5;
m, p and q are each 0 or 1;
W is a D or L isomer of Pro, Arg, HArg,.N-MeArg, Lys or Orn;
X is a D or L isomer of Arg, Lys, Orn or Gln; or when m and q are 1, X may be Gly;
Y is a D or L isomer of Arg, Lys, Orn, Ser, Gln, Tyr or Ala;
Z is $NH(CH_2)_t$-NHR; a D or L isomer of Arg-$NH_2$, Lys-$NH_2$ or Orn-$NH_2$; or, when at least one of m, p and q is 1, NHR′ or OH;
R is H or C(=NH)-$NH_2$;
R′ is H or Alk;
t is 2-6; and
Alk is alkyl having 1-4 carbon atoms;
or a pharmaceutically acceptable acid addition salt, or lower alkyl or benzyl ester thereof; which comprises

a) (i) for compounds in which Z is other than NH-$(CH_2)_t$-NHR, cyclising by means of two mercapto groups, an optionally protected compound of Formula II:

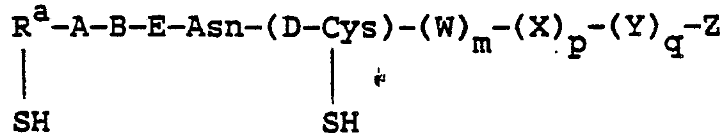

(II)

in which $R^a$ is β-mercapto-β,β-cyclopenta(or cyclotetra)methylenepropionic acid and A, B, E, W, X, Y, Z, m, p and q are as defined in claim 1, with the mercapto groups being members of the $R^a$ and D-Cys units; or

(ii) for compounds in which Z is NH-$(CH_2)_t$-NHR, reacting a compound of the formula:

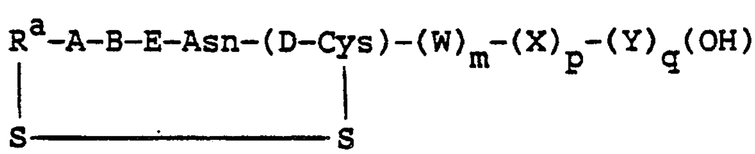

with a compound of the formula: $NH_2$-$(CH_2)_t$-NHR in which $R^a$ is as hereinbefore defined and A, B, E, W, X, Y, m, p, q, t and R are as defined in claim 1;

b) removing any protecting groups, if necessary;

c) optionally forming a lower alkyl or benzyl ester; and

d) optionally forming a pharmaceutically acceptable acid addition salt.

2. A process according to claim 1 in which A is D-Tyr or D-Tyr(Et); B is Phe; E is Val; m and p are 1 and q is 0; W is a D or L isomer of Pro, Arg or N-MeArg; X is a D or L isomer of Arg or Gly; and Z is NHR or a D or L isomer of Arg-$NH_2$.

3. A process according to claim 1 or 2 in which $(W)_m$-$(X)_p$-$(Y)_q$-Z is Pro-Arg-$NH_2$, D-Arg-Arg-$NH_2$, Arg Arg-$NH_2$, Arg-D Arg-$NH_2$, D-Arg-D-Arg-$NH_2$, or N-MeArg-Arg-$NH_2$.

4. A process according to claim 1 in which $(W)_m$-$(X)_p$-$(Y)_q$-Z is Pro-NH$(CH_2)_4$NHC(=NH)-$NH_2$ Pro-Arg-NH$(CH_2)_2$$NH_2$, Arg-$NH_2$ or Arg-Gly-Arg-$NH_2$.

5. A process according to claim 2 in which $(W)_m$-$(X)_p$-$(Y)_q$-Z is Pro-Arg-$NH_2$, D-Arg-Arg-$NH_2$ or Arg-D-Arg-$NH_2$.

6. A process according to claim 1 for preparing: [1-(β-mercapto-β,β-cyclopentamethylenepropionic acid)-2-(O-ethyl)-D-tyrosine-4-valine 6-D-cysteine-7-D-arginine-9-desglycine]vasopressin; [1-(β-mercapto-β,β-cyclopentamethylenepropionic acid)-2-(O-ethyl)-D-tyrosine-4-valine-6-D-cysteine-9-desglycine]vasopressin; or [1-(β-mercapto β,β-cyclopentamethylenepropionic acid)-2-(O-ethyl)-D-tyrosine-4-valine-6-D-cysteine-7-arginine-8-D-arginine-9-desglycine]vasopressin.

7. The use of a compound according to formula (I) as defined in claim 1, or a pharmaceutically acceptable salt thereof, in the manufacture of a medicament.

8. A process for preparing a pharmaceutical composition which comprises bringing into association a compound of the formula (I) or a pharmaceutically acceptable salt thereof as defined in claim 1 and a pharmaceutically acceptable carrier.

9. A process for preparing a compound of formula (II):

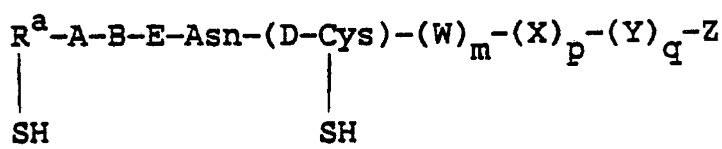

(II)

wherein $R^a$, A, B, E, W, X, Y, Z, m, p and q are as defined in claim 1, which comprises

a) coupling suitably protected amino acids to a benzhydrylamine or chloromethyl resin;

b) splitting the peptide from the carrying resin matrix; and

c) optionally removing the protective groups, if present.

Claims for the following Contracting State: (GR):

1. A process for preparing a compound of the formula (I):

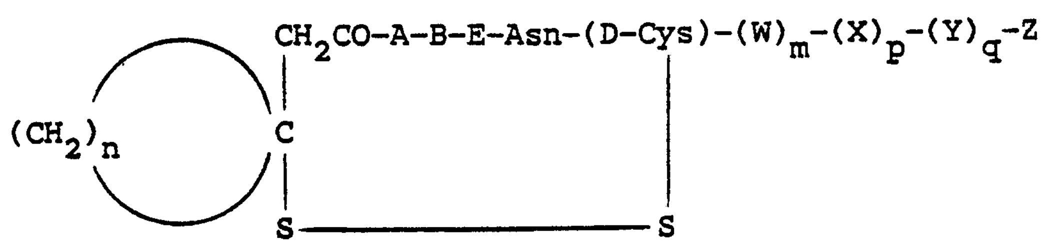

(I)

in which:

A is a D or L isomer of Phe, Phe(4′-Alk), Ile, Cha, Tyr, or Tyr(O-Alk);

B is Phe, Phe(4′-Alk), Tyr(O-Alk), Ile or Tyr;

E is Val, Ile, Abu, Chg, Gln, Lys, Cha, Nle, Leu, Ala or Gly;

n is 4 or 5;
m, p and q are each 0 or 1;
W is a D or L isomer of Pro, Arg, HArg, N-MeArg, Lys or Orn;
X is a D or L isomer of Arg, Lys, Orn or Gln; or when m and q are 1, X may be Gly;
Y is a D or L isomer of Arg, Lys, Orn, Ser, Gln, Tyr or Ala;
Z is NH-$(CH_2)_t$-NHR; a D or L isomer of Arg-$NH_2$, Lys-$NH_2$ or Orn-$NH_2$; or, when at least one of m, p and q is 1, NHR′ or OH;
R is H or C(=NH)-$NH_2$;
R′ is H or Alk;
t is 2-6; and
Alk is alkyl having 1-4 carbon atoms;
or a pharmaceutically acceptable acid addition salt, or lower alkyl or benzyl ester thereof; which comprises

a) (i) for compounds in which Z is other than NH-$(CH_2)_t$-NHR, cyclising by means of two mercapto groups, an optionally protected compound of Formula II:

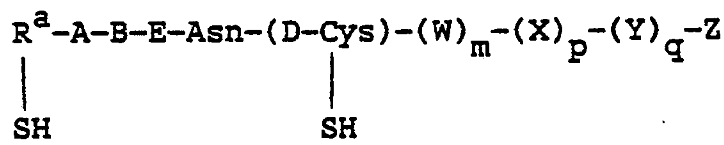

(II)

in which $R^a$ is β-mercapto-β,β-cyclopenta(or cyclotetra)methylenepropionic acid and A, B, E, W, X, Y, Z, m, p and q are as defined in claim 1, with the mercapto groups being members of the $R^a$ and D-Cys units; or

(ii) for compounds in which Z is NH-$(CH_2)_t$-NHR, reacting a compound of the formula:

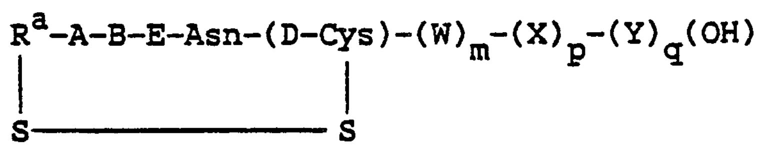

with a compound of the formula: $NH_2$-$(CH_2)_t$-NHR in which $R^a$ is as hereinbefore defined and A, B, E, W, X, Y, m, p, q, t and R are as defined in claim 1;

b) removing any protecting groups, if necessary;

c) optionally forming a lower alkyl or benzyl ester; and

d) optionally forming a pharmaceutically acceptable acid addition salt.

2. A process according to claim 1 in which A is D-Tyr or D-Tyr(Et); B is Phe; E is Val; m and p are 1 and q is 0; W is a D or L isomer of Pro, Arg or N-MeArg; X is a D or L isomer of Arg or Gly; and Z is NHR or a D or L isomer of Arg-$NH_2$.

3. A process according to claim 1 or 2 in which $(W)_m$-$(X)_p$-$(Y)_q$-Z is Pro-Arg-$NH_2$, D-Arg-Arg-$NH_2$, Arg-Arg-$NH_2$, Arg-D-Arg-$NH_2$, D-Arg-D-Arg $NH_2$, or N-MeArg-Arg-$NH_2$.

4. A process according to claim 1 in which $(W)_m$-$(X)_p$-$(Y)_q$-Z is Pro-$NH(CH_2)_4NHC$(=NH)-$NH_2$, Pro-Arg-$NH(CH_2)_2NH_2$, Arg-$NH_2$ or Arg-Gly-Arg-$NH_2$.

5. A process according to claim 2 in which $(W)_m$__$(X)_p$-$(Y)_q$-Z is Pro-Arg-$NH_2$, D-Arg-Arg-$NH_2$ or Arg-D-Arg-$NH_2$.

6. A process according to claim 1 for preparing [1-(β-mercapto-β,β-cyclopentamethylenepropionic acid)-2-(O-ethyl)-D-tyrosine-4-valine-6-D-cysteine-7-D-arginine-9-desglycine]vasopressin;
[1-(β-mercapto-β,β-cyclopentamethylenepropionic acid)-2-(O-ethyl)-D-tyrosine-4-valine-6-D-cysteine-9-desglycine]vasopressin; or
[1-(β-mercapto-β,β-cyclopentamethylenepropionic acid)-2-(O-ethyl)-D-tyrosine-4-valine-6-D-cysteine-7-arginine-8-D-arginine-9-desglycine]vasopressin.

7. The use of a compound according to formula (I) as defined in claim 1, or a pharmaceutically acceptable salt thereof, in the manufacture of a medicament.

8. A process for preparing a pharmaceutical composition which comprises bringing into association a compound of the formula (I) or a pharmaceutically acceptable salt thereof as defined in claim 1 and a pharmaceutically acceptable carrier.

9. An intermediate compound of formula (II):

$$\underset{\underset{\mathrm{SH}}{|}}{R^{a}}\text{-A-B-E-Asn-(D-}\underset{\underset{\mathrm{SH}}{|}}{\text{Cys}}\text{)-(W)}_{m}\text{-(X)}_{p}\text{-(Y)}_{q}\text{-Z}$$

wherein:
$R^a$ is $\beta$-mercapto-$\beta,\beta$-cyclopenta(or cyclotetra)methylenepropionic acid and A, B, E, W, X, Y, Z, m, p and q are as defined in claim 1.

10. A process for preparing a compound of formula (II):

$$\underset{\underset{\mathrm{SH}}{|}}{R^{a}}\text{-A-B-E-Asn-(D-}\underset{\underset{\mathrm{SH}}{|}}{\text{Cys}}\text{)-(W)}_{m}\text{-(X)}_{p}\text{-(Y)}_{q}\text{-Z}$$

(II)

wherein $R^a$, A, B, E, W, X, Y, Z, m, p and q are as defined in claim 1, which comprises

a) coupling suitably protected amino acids to a benzhydrylamine or chloromethyl resin;

b) splitting the peptide from the carrying resin matrix; and

c) optionally removing the protective groups, if present.